# EUROPÄISCHE PATENTSCHRIFT

(11) **EP 2 046 754 B1**
(45) Veröffentlichungstag und Bekanntmachung des Hinweises auf die Patenterteilung: **21.04.2010**
(21) Anmeldenummer: 07786009.6
(22) Anmeldetag: 12.07.2007
(51) Int. Cl.: C07D 231/14

(54) **VERFAHREN ZUM HERSTELLEN VON ALKYLANILIDEN AUS HALOGENBENZOLDERIVATEN**
METHOD FOR PRODUCING ALKYLANILIDES FROM HALOBENZENE DERIVATIVES
PROCEDE DESTINE À PRODUIRE DES AKLYLANILIDES À PARTIR DE DERIVES D'HALOGENOBENZENES

(30) Priorität: 14.07.2006 DE 102006033090
(43) Veröffentlichungstag der Anmeldung: 15.04.2009
(73) Patentinhaber: Bayer CropScience AG, 40789 Monheim (DE)
(72) Erfinder: STRAUB, Alexander, 42117 Wuppertal (DE)
(86) Internationale Anmeldenummer: PCT/EP2007/006177
(87) Internationale Veröffentlichungsnummer: WO 2008/006575

(56) Entgegenhaltungen:
- EP-A- 0 824 099
- WO-A-03/074491
- WO-A-2004/103975
- WO-A-2005/042492
- WO-A-2006/061226
- ARTIS KLAPARS ET AL: "A General and Efficient Copper Catalyst for the Amidation of Aryl Halides and the N-Arylation of Nitrogen Heterocycles" JOURNAL OF THE AMERICAN CHEMICAL SOCIETY, AMERICAN CHEMICAL SOCIETY, WASHINGTON, DC, US, Bd. 123, 7. Dezember 2001 (2001-12-07), Seiten 7727-7729, XP002211988 ISSN: 0002-7863
- KANG S-K ET AL: "COPPER-CATALYZED N-ARYLATION OF ARYL IODIDES WITH BENZAMIDES OR NITROGEN HETEROCYLCES IN THE PRESENCE OF ETHYLENEDIAMINE" SYNLETT, THIEME INTERNATIONAL, STUTTGART, DE, Bd. 3, 4. März 2002 (2002-03-04), Seiten 427-430, XP001156275 ISSN: 0936-5214
- YANG B H ET AL: "Palladium-catalyzed amination of aryl halides and sulfonates" JOURNAL OF ORGANOMETALLIC CHEMISTRY, ELSEVIER-SEQUOIA S.A. LAUSANNE, CH, Bd. 576, Nr. 1-2, 15. März 1999 (1999-03-15), Seiten 125-146, XP004166280 ISSN: 0022-328X
- JINGJUN YIN ET AL: "PALLADIUM-CATALYZED INTERMOLECULAR COUPLING OF ARYL HALIDES AND AMIDES" ORGANIC LETTERS, ACS, WASHINGTON, DC, US, Bd. 2, Nr. 8, 2000, Seiten 1101-1104, XP002413650 ISSN: 1523-7060
- WOLFE J P ET AL: "Nickel-Catalyzed Amination of Aryl Chlorides" JOURNAL OF THE AMERICAN CHEMICAL SOCIETY, AMERICAN CHEMICAL SOCIETY, WASHINGTON, DC, US, Bd. 119, Nr. 26, 1997, Seiten 6054-6058, XP002442784 ISSN: 0002-7863

## Beschreibung

Die vorliegende Erfindung betrifft ein Verfahren zum Herstellen von bekannten fungizid wirksamen Alkylaniliden aus 2-Alkylhalogenaromaten und Heterocyclylamiden.

Aus EP-A-0 824 099 und WO-A-03010149 ist bekannt, dass man Alkylanilide durch Umsetzung des entsprechenden Säurechlorids mit dem entsprechenden Alkylanilinderivat erhält.

Sowohl Darstellung und Handhabung der Säurechloride, als auch die Darstellung der Alkylanilide sind mit erheblichem technischem Aufwand verbunden. So müssen die Säurechloride beispielsweise vor der Reaktion durch einen zeit- und kostenintensiven Destillationsschritt aufgereinigt werden.

Die Darstellung der Aniline erfolgt üblicherweise, wie in WO-A-3074491 beschrieben, durch eine aufwändige Synthese aus den entsprechenden Bromaromaten und Benzophenonimin oder bei Temperaturen von 150 °C und Drucken von 75 bis 85 bar mit Ammoniakgas, wie in WO-A-06061226 beschrieben.

Alkylaniline zeigen jedoch häufig toxische Eigenschaften und sind potentiell mutagen.

Im Stand der Technik ist bekannt, dass man Arylhalogenide mit Amiden unter Palladium-oder Kupfer-Katalyse zu Alkylaniliden umsetzen kann (J. Am. Chem. Soc. 2002, 124, 7420).

Häufig findet man jedoch, dass Metall-katalysierte Reaktionen mit Heterocyclen, die als chelatisierende Liganden fungieren können, inhibiert sind. Darüber hinaus sind orthosubstituierte Halogenaromaten sterisch für einen Halogenaustausch gehindert.

Der vorliegenden Erfindung liegt daher die Aufgabe zugrunde, ein Verfahren zur einfachen und selektiven Herstellung von Alkylaniliden bereitzustellen, das die im Stand der Technik beschriebenen Nachteile nicht aufweist.

Überraschenderweise wurden Bedingungen gefunden, unter denen sich Heterocyclylamide mit ortho-substituierten Halogenaromaten gut umsetzen lassen.

Gegenstand der vorliegenden Erfindung ist also ein Verfahren zum Herstellen von Alkylaniliden der Formel (I) 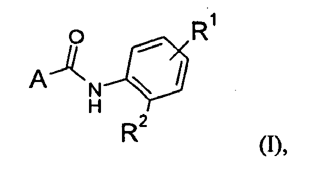 in welcher
- R¹: für Wasserstoff, Halogen, -CR' (R' = Wasserstoff, Fluor oder O-C₁₋₄-Alkyl), besonders bevorzugt für Wasserstoff steht;
- R²: für -CH(Me)-CH₂-CHMe₂, -CH₂-CH₂-t-But, oder 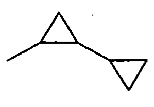 steht, wobei - CH(Me)-CH₂-CHMe₂ und 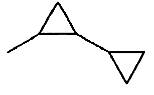 besonders bevorzugt sind,
- A: für den Rest der Formel (A1) steht, 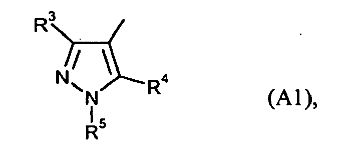
in welcher
- R³: für Wasserstoff, Cyano, Halogen, Nitro, C₁-C₄-Alkyl, C₁-C₄-Alkoxy, C₁-C₄-Alkylthio, C₃-C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht;
- R⁴: für Wasserstoff, Halogen, Cyano, C₁-C₄-Alkyl, C₁-C₄-Alkoxy oder C₁-C₄-Alkylthio steht;
- R⁵: für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht;
oder
- A: für den Rest der Formel (A2) steht, 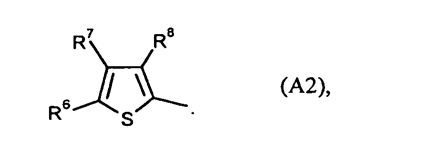
in welcher
- R⁶ und R⁷: unabhängig voneinander für Wasserstoff, Halogen, C₁-C₄-Alkyl oder C₁-C₄-Hatogenalkyl mit 1 bis 5 Halogenatomen stehen,
- R⁸: für Halogen, Cyano oder C₁-C₄-Alkyl, oder C₁-C₄-Halogenalkyl oder C₁-C₄-Halo genalkoxy mit jeweils 1 bis 5 Halogenatomen steht,
oder
- A: für den Rest der Formel (A3) steht, 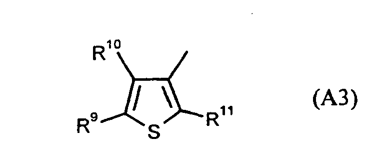
in welcher
- R⁹ und R¹⁰: unabhängig voneinander für Wasserstoff, Halogen, C₁-C₄-Alkyl oder C₁-C₄-Halogenalkyl mit 1 bis 5 Halogenatomen stehen,
- R¹¹: für Wasserstoff, Halogen, C₁-C₄-Alkyl oder C₁-C₄-Halogenalkyl mit 1 bis 5 Halogenatomen steht,
oder
- A: für den Rest der Formel (A4) steht, 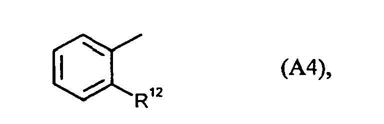
in welcher
- R¹²: für Wasserstoff, Halogen, Hydroxy, Cyano, C₁-C₆-Alkyl, C₁-C₄-Halogenalkyl, C₁-C₄-Halogenalkoxy oder C₁-C₄-Halogenalkylthio mit jeweils 1 bis 5 Halogenatomen steht,
oder
- A: für den Rest der Formel (A5) steht, 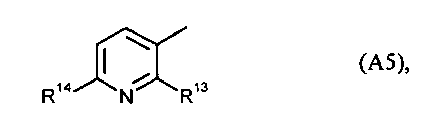
in welcher
- R¹³: für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
- R¹⁴: für Wasserstoff, Halogen, Cyano, C₁-C₄-Alkyl, C₁-C₄-Alkoxy, C₁-C₄-Alkylthio, C₁-C₄-Halogenalkyl, C₁-C₄-Halogenalkoxy mit jeweils 1 bis 5 Halogenatomen, C₁-C₄-Alkylsulphinyl oder C₁-C₄-Alkylsulphonyl steht,
oder
- A: für den Rest der Formel (A6) steht, 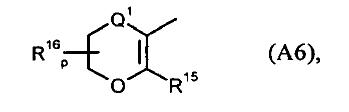
in welcher
- R¹⁵: für C₁-C₄-Alkyl oder C₁-C₄-Halogenalkyl mit 1 bis 5 Halogenatomen steht,
- R¹⁶: für C₁-C₄-Alkyl steht,
- Q¹: für S (Schwefel), O (Sauerstoff), SO, SO₂ oder CH₂ steht,
- p: für 0, 1 oder 2, wobei R¹⁶ für identische oder verschiedene Reste steht, wenn p für 2 steht,
oder
- A: für den Rest der Formel (A7) steht 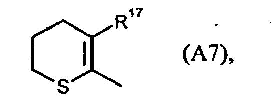
in welcher
- R¹⁷: für C₁-C₄-Alkyl oder C₁-C₄-Halogenalkyl mit 1 bis 5 Halogenatomen steht,
oder
- A: für den Rest der Formel (A8) steht 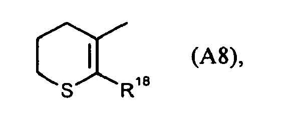
in welcher
- R¹⁸: für C₁-C₄-Alkyl oder C₁-C₄-Halogenalkyl mit 1 bis 5 Halogenatomen steht,
oder
- A: für den Rest der Formel (A9) steht 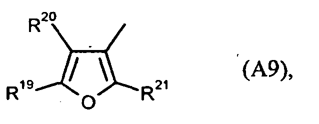
in welcher
- R¹⁹ und R²⁰: unabhängig voneinander für Wasserstoff, Halogen, Amino, C₁-C₄-Alkyl oder C₁-C₄-Halogenalkyl mit 1 bis 5 Halogenatomen stehen,
- R²¹: für Wasserstoff, Halogen, C₁-C₄-Alkyl oder C₁-C₄-Halogenalkyl mit 1 bis 5 Halogenatomen steht,
oder
- A: für den Rest der Formel (A 10) steht 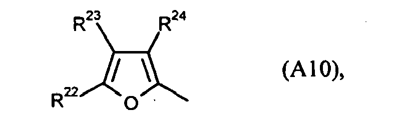
in welcher
- R²² und R²³: unabhängig voneinander für Wasserstoff, Halogen, Amino, Nitro, C₁-C₄-Alkyl oder C₁-C₄-Halogenalkyl having 1 bis 5 Halogenatomen stehen,
- R²⁴: für Wasserstoff, Halogen, C₁-C₄-Alkyl oder C₁-C₄-Halogenalkyl mit 1 bis 5 Halogenatomen steht,
oder
- A: für den Rest der Formel (A11) steht 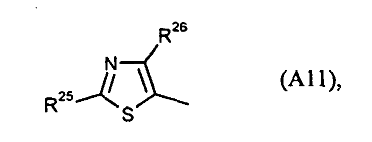
in welcher
- R²⁵: für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
- R²⁶: für Halogen, C₁-C₄-Alkyl oder C₁-C₄-Halogenalkyl mit 1 bis 5 Halogenatomen steht,
oder
- A: für den Rest der Formel (A12) steht 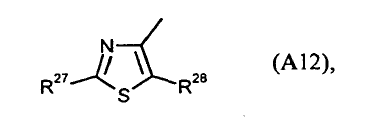
in welcher
- R²⁷: für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
- R²⁸: für Halogen, C₁-C₄-Alkyl oder C₁-C₄-Halogenalkyl mit 1 bis 5 Halogenatomen steht,
oder
- A: für den Rest der Formel (A13) steht 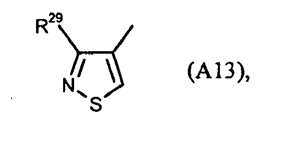
in welcher
- R²⁹: für Halogen, C₁-C₄-Alkyl oder C₁-C₄-Halogenalkyl mit 1 bis 5 Halogenatomen steht,
oder
- A: für den Rest der Formel (A14) steht 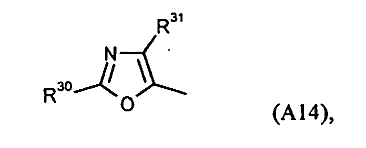
in welcher
- R³⁰: für Wasserstoff oder C₁-C₄-Alkyl steht,
- R³¹: für Halogen oder C₁-C₄-Alkyl steht,
oder
- A: für den Rest der Formel (A15) steht 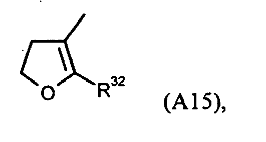
in welcher
- R³²: für C₁-C₄-Alkyl oder C₁-C₄-Halogenalkyl mit 1 bis 5 Halogenatomen steht,
oder
- A: für den Rest der Formel (A16) steht 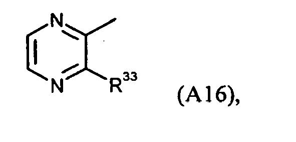
in welcher
- R³³: für Wasserstoff, Halogen, C₁-C₄-Alkyl oder C₁-C₄-Halogenalkyl mit 1 bis 5 Halogenatomen steht,
oder
- A: für den Rest der Formel (A17) steht 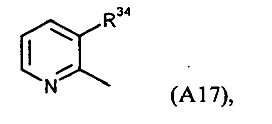
in welcher
- R³⁴: für Halogen, Hydroxy, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
oder
- A: für den Rest der Formel (A18) steht 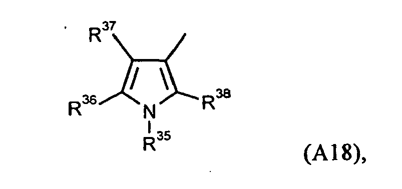
in welcher
- R³⁵: für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄-Alkylsulfonyl, Di(C₁-C₄-alkyl)aminosulfonyl, C₁-C₆-Alkylcarbonyl oder jeweils gegebenenfalls substituiertes Phenylsulfonyl oder Benzoyl steht,
- R³⁶: für Wasserstoff, Halogen, C₁-C₄-Alkyl oder C₁-C₄-Halogenalkyl mit 1 bis 5 Halogenatomen steht,
- R³⁷: für Wasserstoff, Halogen, Cyano, C₁-C₄-Alkyl oder C₁-C₄-Halogenalkyl mit 1 bis 5 Halogenatomen steht,
- R³⁸: für Wasserstoff, Halogen, C₁-C₄-Alkyl oder C₁-C₄-Halogenalkyl mit 1 bis 5 Halogenatomen steht,
oder
- A: für den Rest der Formel (A19) steht 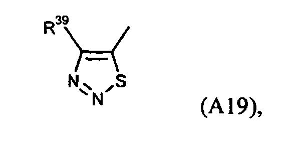
in welcher
- R³⁹: für C₁-C₄-Alkyl steht,
dadurch gekennzeichnet, dass man
in einem ersten Schritt Carbonsäureamide der Formel (II) 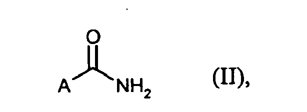 in welcher der Rest A die oben angegebenen Bedeutungen hat,
mit Halogenalkylbenzolen der Formel (III) 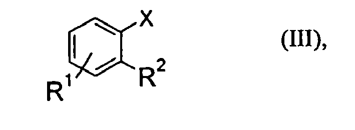 in welcher
die Reste R¹ und R² die oben angegebenen Bedeutungen haben und sich der Substituent R¹ vorzugsweise in meta- oder para-Position, besonders bevorzugt in 4-Position (para zu X) des Aromaten befindet; und
der Rest X ein Halogen, vorzugsweise Cl oder Br, besonders bevorzugt Br ist,
in einer Metall-katalysierten Reaktion umsetzt.

Des Weiteren betrifft die vorliegende Erfindung Verbindungen der Formel (III) 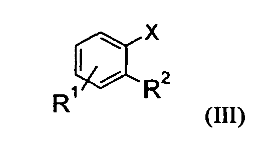 wobei
- R¹: für Wasserstoff, Halogen, -CR'(CF₃)₂ (R'= Wasserstoff, Fluor oder O-C₁₋₄-Alkyl), besonders bevorzugt für Wasserstoff steht und sich der Substituent R¹ vorzugsweise in meta- oder para-Position, besonders bevorzugt in 4-Position (para zu X) des Aromaten befindet; und
- R²: für -CH(Me)-CH₂-CHMe₂ und -CH₂-CH₂-t-But steht,
oder
- R¹: für Halogen, -CR'(CF₃)₂ (R'= Wasserstoff, Fluor oder O-C₁₋₄-Alkyl), besonders bevorzugt für Wasserstoff steht und sich der Substituent R¹ vorzugsweise in meta- oder para-Position, besonders bevorzugt in 4-Position (para zu X) des Aromaten befindet; und
- R²: für 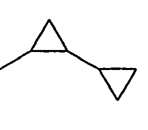 steht
und
- X: ein Halogen, vorzugsweise Cl oder Br, besonders bevorzugt Br ist.

Zudem betrifft die vorliegende Erfindung die Verbindungen der Formeln (IV) bis (VI)

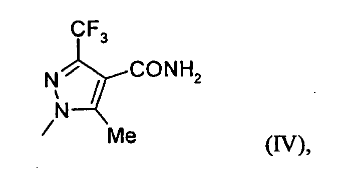

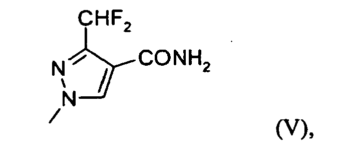

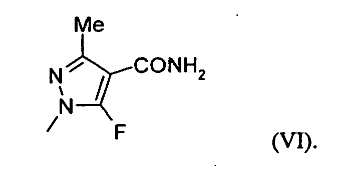

Ein weiterer Aspekt der vorliegenden Erfindung betrifft die Verwendung der Verbindungen gemäß einer der Formeln (II), (III), (IV), (V) und (VI) als Edukte/Zwischenstufen in dem zuvor beschriebenen erfindungsgemäßen Verfahren.

Die erfindungsgemäße Reaktion sei anhand des nachfolgenden Schema (I) näher beschrieben:

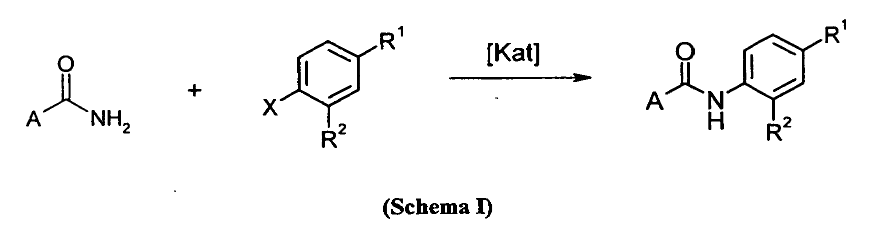

Im Zusammenhang mit der vorliegenden Erfindung umfasst der Begriff Halogen (X) Elemente, die ausgewählt sind aus der Gruppe bestehend aus Fluor, Chlor, Brom und Iod, wobei Chlor und Brom bevorzugt und Brom besonders bevorzugt verwendet wird.

Gegebenenfalls substituierte Reste können einfach oder mehrfach substituiert sein, wobei bei Mehrfachsubstitutionen die Substituenten gleich oder verschieden sein können.

Die Definition C₁-C₆-Alkyl umfasst den größten hierin definierten Bereich für einen Alkylrest. Im Einzelnen umfasst diese Definition die Bedeutungen Methyl, Ethyl, n-, isoPropyl, n-, iso-, sec-, tert-Butyl, sowie jeweils alle isomeren Pentyle und Hexyle.

Die Definition C₂-C₆-Alkenyl umfasst den größten hierin definierten Bereich für einen Alkenylrest. Im Einzelnen umfasst diese Definition die Bedeutungen Ethenyl, n-, isoPropenyl, n-, iso-, sec-, tert-Butenyl, sowie jeweils alle isomeren Pentenyle und Hexenyle.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die benötigten Halogenalkylbenzole sind im Stand der Technik, beispielsweise aus WO-A-03074491 bekannt oder können nach bekannten Methoden durch Friedel-Crafts-Alkylierung oder elektrophile Aromatenhalogenierung erhalten werden.

Eine weitere Möglichkeit besteht in der Umsetzung von 2-Halogenbenzaldehyden mit Wittig-Reagenzien, wobei die so erhalten Propenonderivate, wie in WO-A-03074491 beschrieben, in Cyclopropylverbindungen überführt werden können.

Andere Möglichkeiten sind die Hydroxyalkylierung von Aromaten oder metallierten Aromaten mit Ketonen oder Säurechloriden und deren anschließende Eliminierung bzw. Reduktion. Ausgehend von 1,2-Dibrombenzol sei dies in Schema (II) beispielhaft aufgeführt:

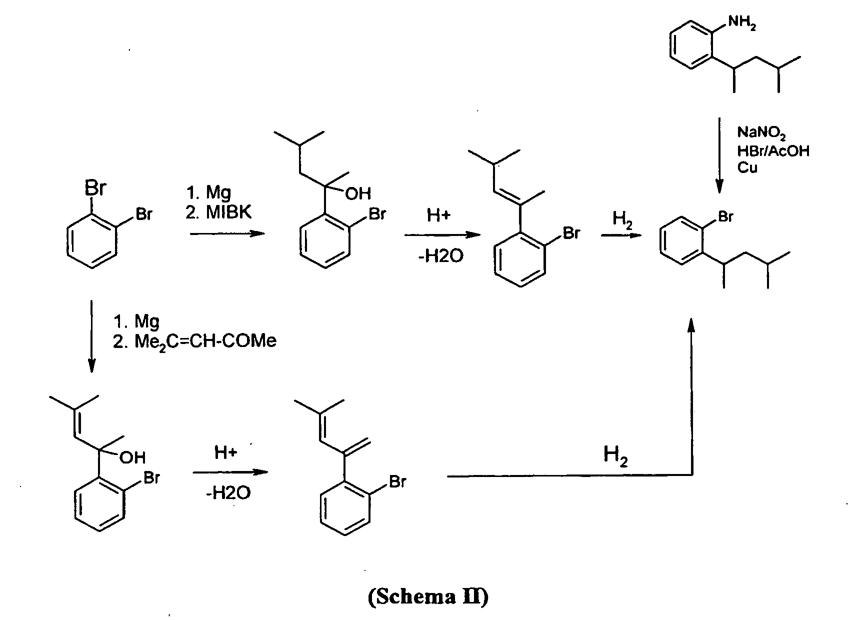

Andererseits kann man auch aus Anilinderivaten durch Diazotierung und Sandmeyer-Reaktion die entsprechenden Halogenverbindungen erhalten.

Gemäß der vorliegenden Erfindung stellt das Halogenalkylbenzol gemäß der Formel (IX) 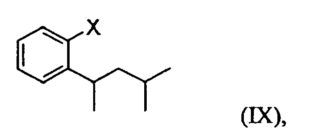 in welcher X für ein Halogenatom, insbesondere für Br steht, ein bevorzugtes Edukt dar.

Die benötigten Säureamide sind nur teilweise bekannt und können aus bekannten Säurehalogeniden, Säuren, Estern oder Nitrilen durch bekannte Reaktionen erhalten werden.

Dies sei durch das folgende Beispiel gemäß Schema (III) verdeutlicht:

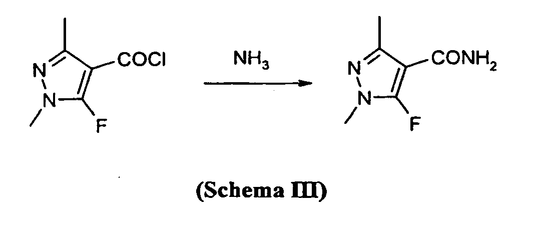

Als Stickstoffquelle kann wässriger oder gasförmiger Ammoniak oder eines seiner Salze, wie z.B. Ammoniumacetat oder Natriumamid, eingesetzt werden.

Als Lösungsmittel für die Herstellung der Säureamide kommen alle Solventien in Frage, die unter den Reaktionsbedingungen stabil sind, wie z.B. Ether wie THF, 2-Methyl-THF, Dioxan, Methyl-t-butylether (MTBE), Diethylenglykol, Diethoxymethan, Dimethoxymethan; aromatische Kohlenwasserstoffe wie Toluol, Xylol, Chlorbenzol, Dichlorbenzol, Benzol; aliphatische Kohlenwasserstoffe wie Petrolether, Heptan, Hexan, Methylcyclohexan, Cyclohexan; Dimethylformamid (DMF); Dimethylacetamid; N-Methylpyrrolidon (NMP); Dimethylsulfoxid (DMSO), Acetonitril; Butyronitril; Wasser; Ketone wie Aceton, Methylisobutylketon (MIBK); Alkohole wie Methanol, Ethanol, Isopropanol.

Für die erfindungsgemäße Umsetzung des Säureamids mit dem Halogenbenzol-Derivat werden Metallkatalysatoren verwendet. Hierfür kommen Palladium und Kupfer in allen Oxidationsstufen, z.B. in metallischer Form oder als Salze in Frage.

Gemäß der vorliegenden Erfindung sind z.B. Pd(OAc)₂, Pd(OH)₂, PdCl₂, Pd(acac)₂ (acac = Acetylacetonat), Pd(NO₃)₂, Pd(dba)₂, Pd₂dba₃, (dba = Dibenzyliden-aceton), Dichloro-bis-(triphenylphosphin)palladium(II), Pd(CH₃CN)₂Cl₂, Tetrakis-(triphenylphosphin)-palladium(0), Pd/C oder Palladiumnanopartikel, CuI, CuCl, CuSCN, Cu₂O, CuO, CuCl₂, CuSO₄, CuBr, CuBr₂, Cu₂S, Cu(OAc)₂, Cu(acac)₂ geeignet, wobei die Kupferverbindungen oder deren Mischungen bevorzugt eingesetzt werden.

Gemäß der vorliegenden Erfindung werden die Katalysatoren in katalytischen Mengen eingesetzt. Dies bedeutet, dass die Metall-Katalysatoren in Konzentrationen von 0,01 bis 50,0 Mol-%, vorzugsweise von 1,0 bis 20,0 Mol%, bezogen auf die Carbonsäureamide der Formel (II), eingesetzt werden.

Die erfindungsgemäße Reaktion wird bevorzugt in Gegenwart von Liganden durchgeführt.

Im Falle der Palladiumkatalyse sind geeignete Liganden beispielsweise ausgewählt aus der Gruppe der Carben- und Phosphinliganden, wobei Xantphos und Tris(t-butyl)phosphin besonders bevorzugt eingesetzt werden.

Im Falle der Kupferkatalyse sind geeignete Liganden z.B. ausgewählt aus der Gruppe bestehend aus Diaminen wie z.B. N,N'-Dimethyl-1,2-cyclohexandiamin (cis oder trans, racemisch oder als Enantiomer), N,N'-Dimethylethylendiamin, Ethylendiamin, N-Methylethylendiamin, N-Butylethylendiamin, N,N,N'-Trimethylethylendiamin oder aber auch 1,10-Phenanthrolin, Ethylendiamintetraessigsäure, Tetra-n-butylammoniumfluorid, tris(3,6-Trioxaheptyl)amin (TDA-1), wobei N,N'-Dimethyl-1,2-cyclohexandiamin besonders bevorzugt eingesetzt wird.

Die Liganden werden dem Metall-Katalysator in einem solchen Verhältnis zugesetzt, dass die gewünschte katalytische Wirkung eintritt.

Gemäß der vorliegenden Erfindung beträgt das Verhältnis von Ligand zu Metall-Katalysator zwischen 0,5 bis 10 Äquivalente, vorzugsweise zwischen 1 bis 5 Äquivalente.

Die Reaktion wird bevorzugt in Gegenwart von Basen durchgeführt. Hierfür kommen z.B. Alkali- und Erdalkalihydroxide wie KOH, NaOH, Ca(OH)₂, -fluoride wie KF, -phosphate wie K₃PO₄, und -carbonate wie Pottasche, Soda, Cäsiumcarbonat oder Phosphazenbasen, Alkoholate wie Natrium-tert-butylat oder Phenolate wie Natriumphenolat in Frage.

Gemäß der vorliegenden Erfindung werden die Basen in Konzentrationen von 0,5 bis 5 Äquivalenten, vorzugsweise von 1,0 bis 3 Äquivalenten, bezogen auf die Carbonsäureamide der Formel (II), eingesetzt.

Die erfindungsgemäße Umsetzung des Säureamids mit dem Halogenbenzolderivat wird vorzugsweise in einem Lösungsmittel durchgeführt.

Als Lösungsmittel verwendet man bevorzugt Dioxan, THF, Diglyme, Toluol, Xylol, DMF.

Gemäß der vorliegenden Erfindung werden die Säureamide und die Halogenbenzolderivate in äquimolarem Verhältnis miteinander umgesetzt. In einer alternativen Ausführungsform kann das Halogenbenzolderivat auch im Überschuss, z.B. als Lösungsmittel, eingesetzt werden.

Die Umsetzung der Säureamide mit den Halogenbenzolderivaten erfolgt erfindungsgemäß bei Temperaturen im Bereich von 20 bis 200 °C, vorzugsweise von 70 bis 150 °C.

In einer besonders bevorzugten Ausführungsform der Erfindung werden Carbonsäureamide gemäß der Formel (V) mit Verbindungen der Formel (X) 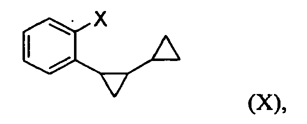 in welcher der Rest X für ein Halogen steht,
zu Carboxamiden der Formel (XI) 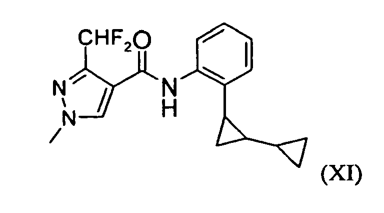 umgesetzt .

Die folgenden Beispiele sollen den Gegenstand der vorliegenden Erfindung näher erläutern, ohne ihn jedoch auf diese zu beschränken.

### Ausführungsbeispiele

### 1-Brom-2-(1,3-dimethylbutyl)benzol

28,4 g 2-(1,3-Dimethylbutyl)anilin werden in 134,9 g (667 mmol) 40-proz. Bromwasserstoffsäure in Eisessig bei 10°C unter Rühren innerhalb von 1,5 h portionsweise mit 12,5 g (181 mmol) Natriumnitrit versetzt. Danach werden 0,5 g Kupferpulver zugegeben und 1 h Stunde unter Rückfluss gekocht. Anschließend werden 120 ml Wasser und Natronlauge bis pH 12 erreicht wird zugegeben, die organische Phase abgetrennt, mit verdünnter Salzsäure gewaschen und im Vakuum eingedampft. Nach Destillation im Kugelrohr bei 0,3 mbar/70°C und Reinigung der Hauptfraktion über präparative HPLC werden 8,3 g eines gelblichen Öls mit einer Reinheit von 99,4 % (bestimmt durch HPLC) erhalten.
¹H-NMR (400 MHz, CDCl₃): 0,89 (dd, 6H), 1,17 (d, 3H), 1,32-1,38 und 1,46-1,53 (2m, AB, 2x1H), 1,54-1,6 (m, 1H), 3,34 (m, 1H), 7 (m, 1H), 7,21-7,28 (m, 2H), 7,52 (d, 1H).

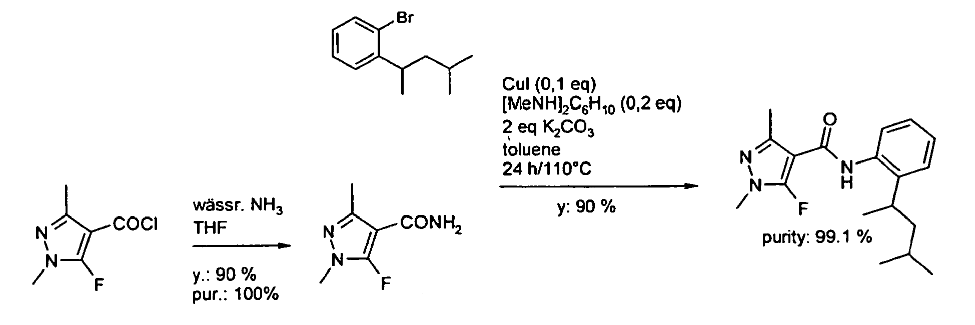

### 5-Fluor-1,3-dimethyl-1H-pyrazol-4-carboxamid

19,9 g 5-Fluor-1,3-dimethyl-1H-pyrazol-4-carbonylchlorid werden bei 20-35°C tropfenweise zu einer Mischung von 35 g 25-proz. Ammoniakwasser und 170 ml THF zugegeben. Nach 4 h Rühren wird der organischen Lösungsmittelanteil im Vakuum eingedampft und 0,2 Mol Pottasche und Kochsalz bis zur Sättigung zugegeben. Nach viermaliger Extraktion mit Essigsäureethylester und Eindampfen der vereinigten organischen Phasen im Vakuum erhält man 16,6 g eines gelben Feststoffs.
¹H-NMR (400 MHz, d⁶-DMSO): 2,64 (s, 3H), 3,63 (s, 3H), 6,9 (broad s, 1H), 7,18 (broad s, 1H).

### N-[2-(1,3-Dimethylbutyl)phenyl]-5-fluor-1,3-dimethyl-1H-pyrazol-4-carboxamid

Eine Mischung aus 0,221 g (1,16 mmol) Kupferiodid, 3,21 g (23,22 mmol) Pottasche und 2,189 g 5-Fluor-1,3-dimethyl-1H-pyrazol-4-carboxamid wird unter Argon mit 330 mg (2,322 mmol) N,N'-Dimethyl-1,2-cyclohexandiamin, 2,8 g (11,6 mmol) 1-Brom-2-(1,3-dimethylbutyl)benzol und 30 ml Toluol versetzt. Nach eintägigem Kochen unter Rückfluss wird die Mischung auf Wasser und 10 ml einer 5% EDTA-Lösung gegossen. Anschließend wird dreimal mit Essigsäureethylester extrahiert und im Vakuum eingedampft. Der ölige Rückstand wird in Toluol gelöst und in n-Hexan eingerührt. Nach Absaugen der ausgefallenen Kristalle werden 3,3 g (89 % der theoretischen Ausbeute) N-[2-(1,3-Dimethylbutyl)phenyl]-5-fluor-1,3-dimethyl-1H-pyrazol-4-carboxamid mit einer Reinheit (HPLC) von 99 % erhalten.

## Patentansprüche

1. Verfahren zur Herstellung von Carboxamiden der allgemeinen Formel (I) 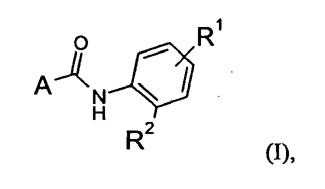 in welcher
R¹ für Wasserstoff, Halogen, -CR' mit R' = Wasserstoff, Fluor oder O-C₁₋₄-Alkyl steht;
R² für -CH(Me)-CH₂-CHN4e₂, -CH₂-CH₂-t-But oder 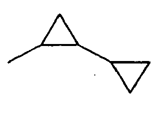 steht,
A für den Rest der Formel (A1) steht, 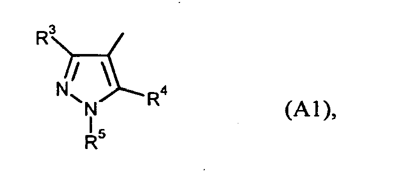
in welcher
R³ für Wasserstoff, Cyano, Halogen, Nitro, C₁-C₄-Alkyl, C₁-C₄-Alkoxy, C₁-C₄-Al-kylthio, C₃-C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht;
R⁴ für Wasserstoff, Halogen, Cyano, C₁-C₄-Alkyl, C₁-C₄-Alkoxy oder C₁-C₄-Alkylthio steht;
R⁵ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht;
oder
A für den Rest der Formel (A2) steht, 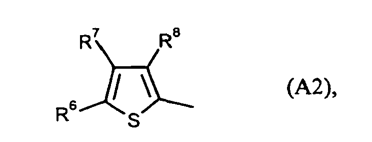
in welcher
R⁶ und R⁷ unabhängig voneinander für Wasserstoff, Halogen, C₁-C₄-Alkyl oder C₁-C₄-Halogenalkyl mit 1 bis 5 Halogenatomen stehen,
R⁸ für Halogen, Cyano oder C₁-C₄-Alkyl, oder C₁-C₄-Halogenalkyl oder C₁- C₄-Halo genalkoxy mit jeweils 1 bis 5 Halogenatomen steht,
oder
A für den Rest der Formel (A3) steht, 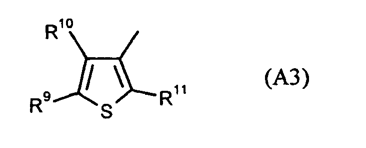
in welcher
R⁹ und R¹⁰ unabhängig voneinander für Wasserstoff, Halogen, C₁-C₄-Alkyl oder C₁-C₄-Halogenalkyl mit 1 bis 5 Halogenatomen stehen,
R¹¹ für Wasserstoff, Halogen, C₁-C₄-Alkyl oder C₁-C₄-Halogenalkyl mit 1 bis 5 Halogenatomen steht,
oder
A für den Rest der Formel (A4) steht, 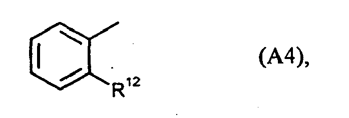
in welcher
R¹² für Wasserstoff, Halogen, Hydroxy, Cyano, C₁-C₆-Alkyl, C₁-C₄-Halogenalkyl, C₁-C₄-Halogenalkoxy oder C₁-C₄-Halogenalkylthio mit jeweils 1 bis 5 Halogenatomen steht,
oder
A für den Rest der Formel (A5) steht, 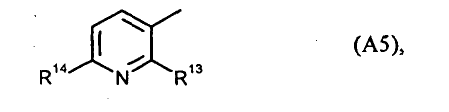
in welcher
R¹³ für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenalk-ylthio oder C₁-C₄-Halogenalkoxy mit jeweils 1 bis 5 Halogenatomen steht,
R¹⁴ für Wasserstoff, Halogen, Cyano, C₁-C₄-Alkyl, C₁-C₄-Alkoxy, C₁-C₄-Alkylthio, C₁-C₄-Halogenalkyl, C₁-C₄-Halogenalkoxy mit jeweils 1 bis 5 Halogenatomen, C₁-C₄-Alkylsulphinyl oder C₁-C₄-Alkylsulphonyl steht,
oder
A für den Rest der Formel (A6) steht, 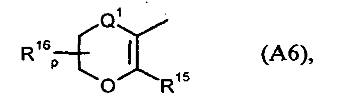
in welcher
R¹⁵ für C₁-C₄-Alkyl oder C₁-C₄-Halogenälkyl mit 1 bis 5 Halogenatomen steht,
R¹⁶ für C₁-C₄-Alkyl steht,
Q¹ für S (Schwefel), O (sauerstoff), SO, SO₂ oder CH₂ steht,
p für 0, 1 oder 2, wobei R¹⁶ für identische oder verschiedene Reste steht, wenn p für 2 steht,
oder
A für den Rest der Formel (A7) steht 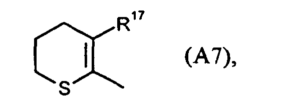
in welcher
R¹⁷ für C₁-C₄-Alkyl oder C₁-C₄-Halogenalkyl mit 1 bis 5 Halogenatomen steht,
oder
A für den Rest der Formel (A8) steht 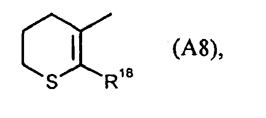
in welcher
R¹⁸ für C₁-C₄-Alkyl oder C₁-C₄-Halogenalkyl mit 1 bis 5 Halogenatomen steht,
oder
A für den Rest der Formel (A9) steht 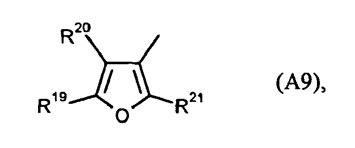
in welcher
R¹⁹ und R²⁰ unabhängig voneinander für Wasserstoff, Halogen, Amino, C₁-C₄-Alkyl oder C₁-C₄-Halogenalkyl mit 1 bis 5 Halogenatomen stehen,
R²¹ für Wasserstoff, Halogen, C₁-C₄-Alkyl oder C₁-C₄-Halogenalkyl mit 1 bis 5 Halogenatomen steht,
oder
A für den Rest der Formel (A10) steht 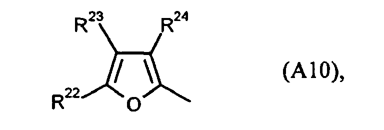 in welcher
R²² und R²³ unabhängig voneinander für Wasserstoff, Halogen, Amino, Nitro, C₁-C₄-Alkyl oder C₁-C₄-Halogenalkyl having 1 bis 5 Halogenatomen stehen,
R²⁴ für Wasserstoff, Halogen, C₁-C₄-Alkyl oder C₁-C₄-Halogenalkyl mit 1 bis 5 Halogenatomen steht,
oder
A für den Rest der Formel (A11) steht 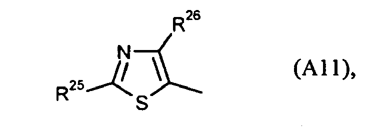
in welcher
R²⁵ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
R²⁶ für Halogen, C₁-C₄-Alkyl oder C₁-C₄-Halogenalkyl mit 1 bis 5 Halogenatomen steht,
oder
A für den Rest der Formel (A 12) steht 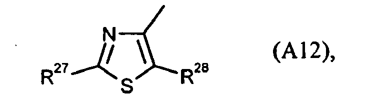
in welcher
R²⁷ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
R²⁸ für Halogen, C₁-C₄-Alkyl oder C₁-C₄-Halogenalkyl mit 1 bis 5 Halogenatomen steht,
oder
A für den Rest der Formel (A13) steht 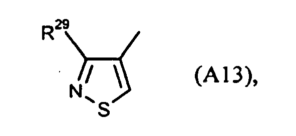
in welcher
R²⁹ für Halogen, C₁-C₄-Alkyl oder C₁-C₄-Halogenalkyl mit 1 bis 5 Halogenatomen steht,
oder
A für den Rest der Formel (A14) steht 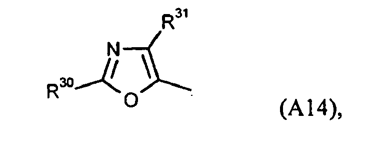
in welcher
R³⁰ für Wasserstoff oder C₁-C₄-Alkyl steht,
R³¹ für Halogen oder C₁-C₄-Alkyl steht,
oder
A für den Rest der Formel (A15) steht 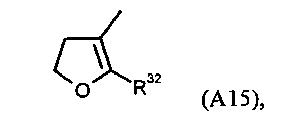
in welcher
R³² für C₁-C₄-Alkyl oder C₁-C₄-Halogenalkyl mit 1 bis 5 Halogenatomen steht,
oder
A für den Rest der Formel (A16) steht 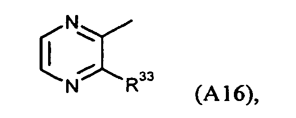
in welcher
R³³ für Wasserstoff, Halogen, C₁-C₄-Alkyl oder C₁-C₄-Halogenalkyl mit 1 bis 5 Halogenatomen steht,
oder
A für den Rest der Formel (A17) steht 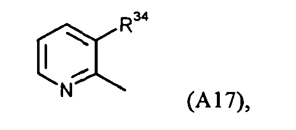 in welcher
R³⁴ für Halogen, Hydroxy, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
oder
A für den Rest der Formel (A18) steht 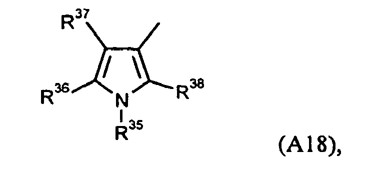
in welcher
R³⁵ für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄-Alkylsulfonyl, Di(C₁-C₄-alkyl)aminosulfonyl, C₁-C₆-Alkylearbonyl oder jeweils gegebenenfalls substituiertes Phenylsulfonyl oder Benzoyl steht,
R³⁶ für Wasserstoff, Halogen, C₁-C₄-Alkyl oder C₁-C₄-Halogenalkyl mit 1 bis 5 Halogenatomen steht,
R³⁷ für Wasserstoff, Halogen, Cyano, C₁-C₄-Alkyl oder C₁-C₄-Halogenalkyl mit 1 bis 5 Halogenatomen steht,
R³⁸ für Wasserstoff, Halogen, C₁-C₄-Alkyl oder C₁-C₄-Halogenalkyl mit 1 bis 5 Halogenatomen steht,
oder
A für den Rest der Formel (A19) steht 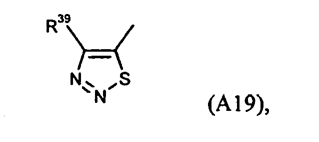
in welcher
R³⁹ für C₁-C₄-Alkyl steht,
**dadurch gekennzeichnet, dass** man
in einem ersten Schritt Carbonsäureamide der Formel (II) 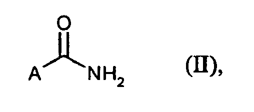 in welcher der Rest A die oben angegebenen Bedeutungen hat
mit Halogenalkylbenzolen der Formel (III) 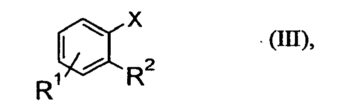 in welcher die Reste R¹ und R² die oben angegebenen Bedeutungen haben und der Rest X ein Halogenatom ist,
in einer Metall-katalysierten Reaktion umsetzt.

2. Verfahren gemäß Anspruch 1, wobei Carbonsäureamide der Formel (II), die ausgewählt sind aus der Gruppe bestehend aus Verbindungen der Formeln (IV) bis (VI) 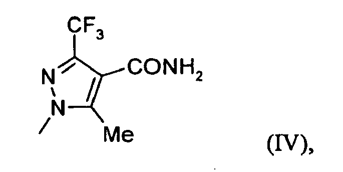 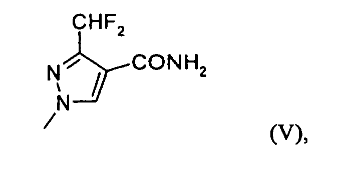 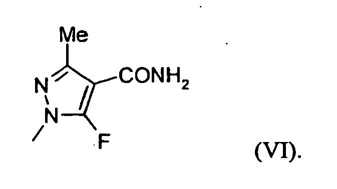 zu Carboxamiden gemäß der allgemeinen Formel (VIII) 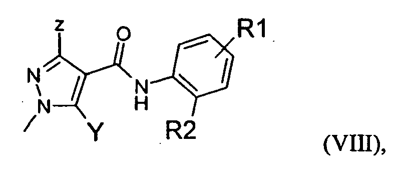 in welcher die Reste R¹ und R² die in Anspruch 1 definierte Bedeutung haben,
Z für Methyl, Difluormethyl oder Trifluormethyl steht und
Y für Wasserstoff, Methyl oder Fluor steht, umgesetzt werden.

3. Verfahren gemäß einem der Ansprüche 1 oder 2, **dadurch gekennzeichnet, dass** Carbonsäureamide gemäß der Formel (V) 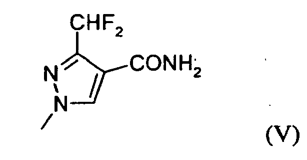 mit Verbindungen der Formel (X) 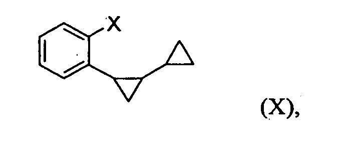 in welcher der Rest X für ein Halogenatom steht,
zu einem Carboxamid der Formel (XI) 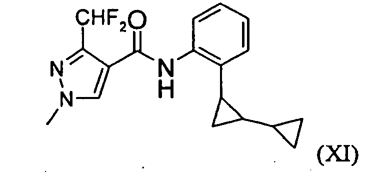 umgesetzt werden.

4. Verfahren gemäß einem der Ansprüche 1 bis 3, **dadurch gekennzeichnet, dass** der Metallkatalysator ausgewählt ist aus Palladium- oder Kupfer-Katalysatoren.

5. Verfahren gemäß einem der Ansprüche 1 bis 4, **dadurch gekennzeichnet, dass** wenigstens eine kupferhaltige Verbindung in katalytischen Mengen in Gegenwart eines Liganden eingesetzt wird.

6. Verfahren gemäß einem der Ansprüche 1 bis 5 unter Verwendung einer Base.

7. Verfahren gemäß Anspruch 5, wobei als Liganden Diamine eingesetzt werden.

8. Verbindung der Formel (III) 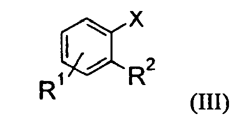 in welcher
R¹ für Wasserstoff, Halogen, -CR'(CF₃)₂ mit R'= Wasserstoff, Fluor oder O-C₁₋₄-Alkyl steht, und
R² für -CH(Me)-CH₂-CHMe₂, -CH₂-CH₂-t-But steht
oder
R¹ für Halogen, -CR'(CF₃)₂ mit R'= Wasserstoff, Fluor oder O-C₁₋₄-Alkyl steht, und
R² für 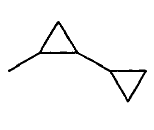 steht
und
X für ein Halogenatom steht.

9. Verbindung gemäß Anspruch 8, der Formel (IX) 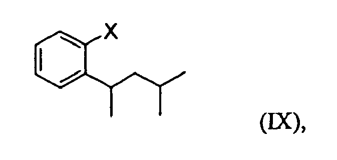 in welcher
X für ein Halogenatom steht.

10. Verbindungen der Formeln (IV) bis (VI)

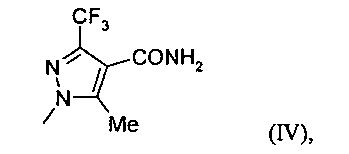

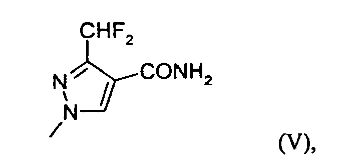

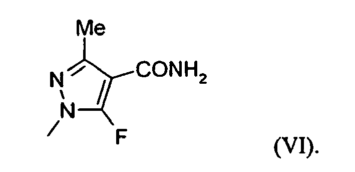

11. Verwendung wenigstens einer Verbindung gemäß einem der Ansprüche 8 bis 10 in einem Verfahren gemäß einem der Ansprüche 1 bis 7.

## Claims

1. Process for preparing carboxamides of the general formula (I) 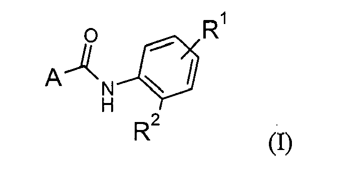 in which
R¹ is hydrogen, halogen, -CR' where R' = hydrogen, fluorine or O-C₁₋₄-alkyl;
R² is -CH(Me)-CH₂-CHMe₂, -CH₂-CH₂-t-But or 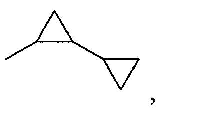
A is the radical of the formula (A1) 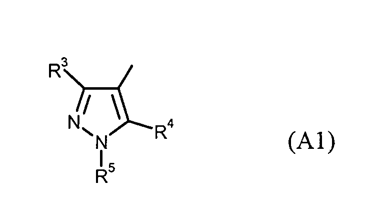
in which
R³ is hydrogen, cyano, halogen, nitro, C₁-C₄-alkyl, C₁-C₄-alkoxy, C₁-C₄-alkylthio, C₃-C₆-cycloalkyl, C₁-C₄-haloalkyl, C₁-C₄-haloalkoxy or C₁-C₄-haloalkylthio having in each case from 1 to 5 halogen atoms, aminocarbonyl or aminocarbonyl-C₁-C₄-alkyl;
R⁴ is hydrogen, halogen, cyano, C₁-C₄-alkyl, C₁-C₄-alkoxy or C₁-C₄-alkylthio;
R⁵ is hydrogen, C₁-C₄-alkyl, hydroxy-C₁-C₄-alkyl, C₂-C₆-alkenyl, C₃-C₆-cycloalkyl, C₁-C₄-alkylthio-C₁-C₄-alkyl, C₁-C₄-alkoxy-C₁-C₄-alkyl, C₁-C₄-haloalkyl, C₁-C₄-haloalkylthio-C₁-C₄-alkyl, C₁-C₄-haloalkoxy-C₁-C₄-alkyl having in each case from 1 to 5 halogen atoms, or phenyl;
or
A is the radical of the formula (A2) 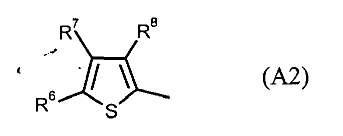 in which
R⁶ and R⁷ are each independently hydrogen, halogen, C₁-C₄-alkyl or C₁-C₄-haloalkyl having from 1 to 5 halogen atoms,
R⁸ is halogen, cyano or C₁-C₄-alkyl, or C₁-C₄-haloalkyl or C₁-C₄-haloalkoxy having in each case from 1 to 5 halogen atoms,
or
A is the radical of the formula (A3) 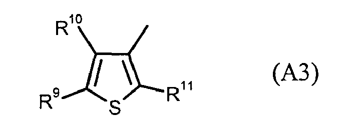
in which
R⁹ and R¹⁰ are each independently hydrogen, halogen, C₁-C₄-alkyl or C₁-C₄-haloalkyl having from 1 to 5 halogen atoms,
R¹¹ is hydrogen, halogen, C₁-C₄-alkyl or C₁-C₄-haloalkyl having from 1 to 5 halogen atoms,
or
A is the radical of the formula (A4) 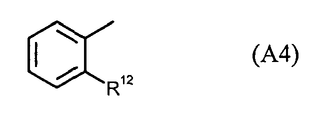
in which
R¹² is hydrogen, halogen, hydroxyl, cyano, C₁-C₆-alkyl, C₁-C₄-haloalkyl, C₁-C₄-haloalkoxy or C₁-C₄-haloalkylthio having in each case from 1 to 5 halogen atoms,
or
A is the radical of the formula (A5) 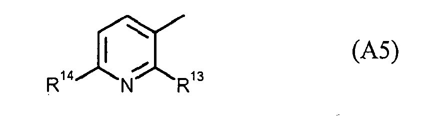
in which
R¹³ is halogen, hydroxyl, cyano, C₁-C₄-alkyl, C₁-C₄-alkoxy, C₁-C₄-alkylthio, C₁-C₄-haloalkyl, C₁-C₄-haloalkylthio or C₁-C₄-haloalkoxy having in each case from 1 to 5 halogen atoms,
R¹⁴ is hydrogen, halogen, cyano, C₁-C₄-alkyl, C₁-C₄-alkoxy, C₁-C₄-alkylthio, C₁-C₄-haloalkyl, C₁-C₄-haloalkoxy having in each case from 1 to 5 halogen atoms, C₁-C₄-alkylsulfinyl or C₁-C₄-alkylsulfonyl,
or
A is the radical of the formula (A6) 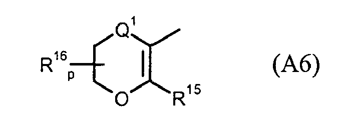
in which
R¹⁵ is C₁-C₄-alkyl or C₁-C₄-haloalkyl having from 1 to 5 halogen atoms,
R¹⁶ is C₁-C₄-alkyl,
Q¹ is S (sulfur), O (oxygen), SO, SO₂ or CH₂,
p is 0, 1 or 2, where R¹⁶ represents identical or different radicals when p is 2,
or
A is the radical of the formula (A7) 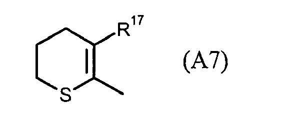
in which
R¹⁷ is C₁-C₄-alkyl or C₁-C₄-haloalkyl having from 1 to 5 halogen atoms,
or
A is the radical of the formula (A8) 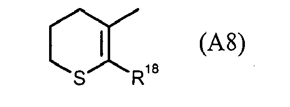
in which
R¹⁸ is C₁-C₄-alkyl or C₁-C₄-haloalkyl having from 1 to 5 halogen atoms,
or
A is the radical of the formula (A9) 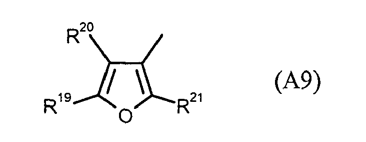
in which
R¹⁹ and R²⁰ are each independently hydrogen, halogen, amino, C₁-C₄-alkyl or C₁-C₄-haloalkyl having from 1 to 5 halogen atoms,
R²¹ is hydrogen, halogen, C₁-C₄-alkyl or C₁-C₄-haloalkyl having from 1 to 5 halogen atoms,
or
A is the radical of the formula (A10) 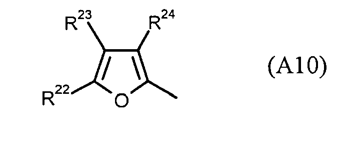
in which
R²² and R²³ are each independently hydrogen, halogen, amino, nitro, C₁-C₄-alkyl or C₁-C₄-haloalkyl having 1 to 5 halogen atoms,
R²⁴ is hydrogen, halogen, C₁-C₄-alkyl or C₁-C₄-haloalkyl having from 1 to 5 halogen atoms,
or
A is the radical of the formula (A11) 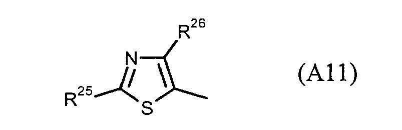
in which
R²⁵ is hydrogen, halogen, amino, C₁-C₄-alkylamino, di(C₁-C₄-alkyl)amino, cyano, C₁-C₄-alkyl or C₁-C₄-haloalkyl having from 1 to 5 halogen atoms,
R²⁶ is halogen, C₁-C₄-alkyl or C₁-C₄-haloalkyl having from 1 to 5 halogen atoms,
or
A is the radical of the formula (A12) 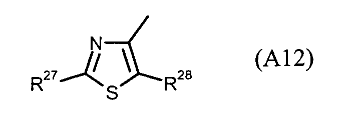
in which
R²⁷ is hydrogen, halogen, amino, C₁-C₄-alkylamino, di(C₁-C₄-alkyl)amino, cyano, C₁-C₄-alkyl or C₁-C₄-haloalkyl having from 1 to 5 halogen atoms,
R²⁸ is halogen, C₁-C₄-alkyl or C₁-C₄-haloalkyl having from 1 to 5 halogen atoms,
or
A is the radical of the formula (A13) 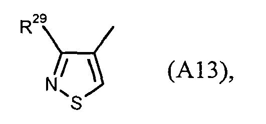
in which
R²⁹ is halogen, C₁-C₄-alkyl or C₁-C₄-haloalkyl having from 1 to 5 halogen atoms,
or
A is the radical of the formula (A14) 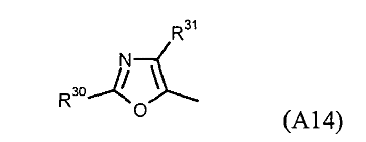
in which
R³⁰ is hydrogen or C₁-C₄-alkyl,
R³¹ is halogen or C₁-C₄-alkyl,
or
A is the radical of the formula (A15) 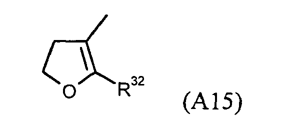
in which
R³² is C₁-C₄-alkyl or C₁-C₄-haloalkyl having from 1 to 5 halogen atoms,
or
A is the radical of the formula (A16) 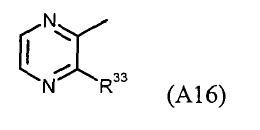
in which
R³³ is hydrogen, halogen, C₁-C₄-alkyl or C₁-C₄-haloalkyl having from 1 to 5 halogen atoms,
or
A is the radical of the formula (A17) 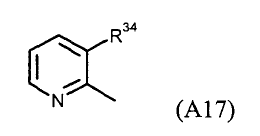
in which
R³⁴ is halogen, hydroxyl, C₁-C₄-alkyl, C₁₋C₄-alkoxy, C₁-C₄-alkylthio, C₁-C₄-haloalkyl, C₁-C₄-haloalkylthio or C₁-C₄-haloalkoxy having in each case from 1 to 5 halogen atoms,
or
A is the radical of the formula (A18) 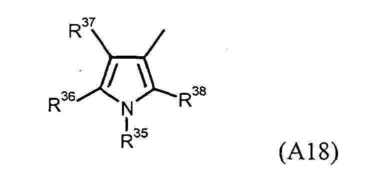
in which
R³⁵ is hydrogen, cyano, C₁-C₄-alkyl, C₁-C₄-haloalkyl having from 1 to 5 halogen atoms, C₁-C₄-alkoxy-C₁-C₄-alkyl, hydroxy-C₁-C₄-alkyl. C₁-C₄-alkylsulfonyl, di(C₁-C₄-alkyl)aminosulfonyl, C₁-C₆-alkylcarbonyl or in each case optionally substituted phenylsulfonyl or benzoyl,
R³⁶ is hydrogen, halogen, C₁-C₄-alkyl or C₁-C₄-haloalkyl having from 1 to 5 halogen atoms,
R³⁷ is hydrogen, halogen, cyano, C₁-C₄-alkyl or C₁-C₄-haloalkyl having from 1 to 5 halogen atoms,
R³⁸ is hydrogen, halogen, C₁-C₄-alkyl or C₁-C₄-haloalkyl having from 1 to 5 halogen atoms,
or
A is the radical of the formula (A19) 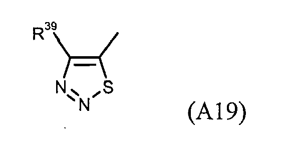
in which
R³⁹ is C₁-C₄-alkyl,
**characterized in that**,
in a first step, carboxamides of the formula (II) 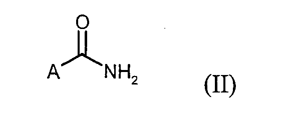 in which the A radical is as defined above
are reacted with haloalkylbenzenes of the formula (III) 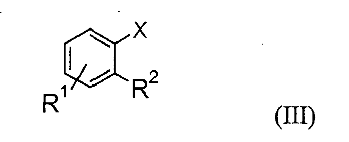 in which the R¹ and R² radicals are each as defined above and the X radical is a halogen atom
in a metal-catalyzed reaction.

2. Process according to Claim 1, wherein carboxamides of the formula (II) which are selected from the group consisting of compounds of the formulae (IV) to (VI) 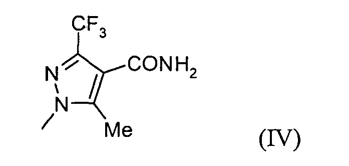 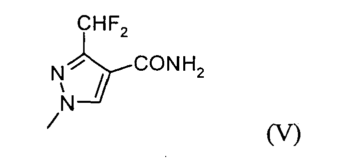 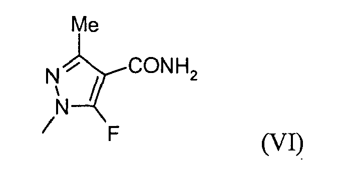 are converted to carboxamides of the general formula (VIII) 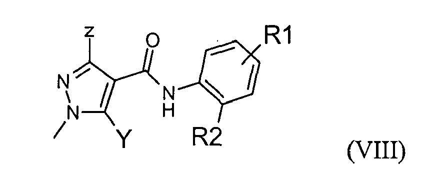 in which the R¹ and R² radicals are each as defined in Claim 1,
Z is methyl, difluoromethyl or trifluoromethyl and
Y is hydrogen, methyl or fluorine.

3. Process according to either of Claims 1 or 2, **characterized in that** carboxamides of the formula (V) 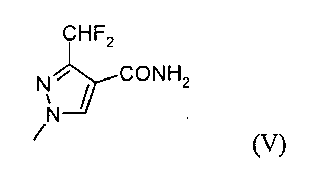 are reacted with compounds of the formula (X) 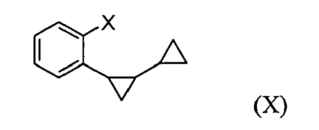 in which the X radical is a halogen atom
to give a carboxamide of the formula (XI)

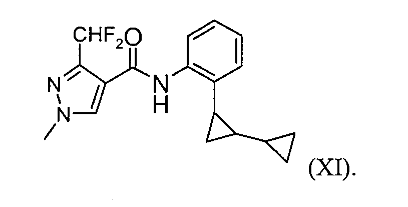

4. Process according to any one of Claims 1 to 3, **characterized in that** the metal catalyst is selected from palladium and copper catalysts.

5. Process according to any one of Claims 1 to 4, **characterized in that** at least one copper compound in catalytic amounts is used in the presence of a ligand.

6. Process according to any one of Claims 1 to 5 using a base.

7. Process according to Claim 5, wherein the ligands used are diamines.

8. Compound of the formula (III) 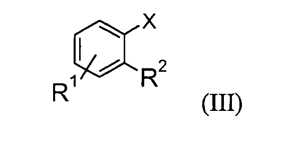 in which
R¹ is hydrogen, halogen, -CR'(CF₃)₂ where R'= hydrogen, fluorine or O-C₁-₄-alkyl, and
R² is -CH(Me)-CH₂-CHMe₂, -CH₂-CH₂-t-But
or
R¹ is halogen, -CR'(CF₃)₂ where R'= hydrogen, fluorine or O-C₁₋₄-alkyl, and
R² is 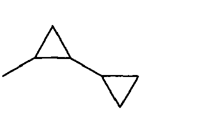 and
X is a halogen atom.

9. Compound according to Claim 8 of the formula (IX) 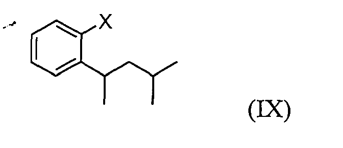 in which
X is a halogen atom.

10. Compounds of the formulae (IV) to (VI)

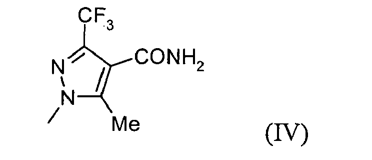

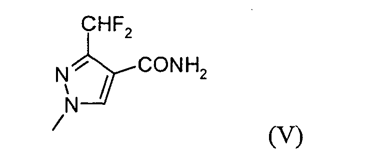

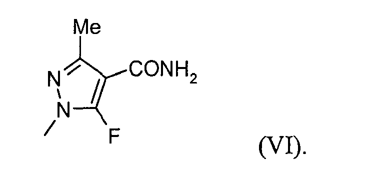

11. Use of at least one compound according to any one of Claims 8 to 10 in a process according to any one of Claims 1 to 7.

## Revendications

1. Procédé pour la préparation de carboxamides de la formule générale (I) 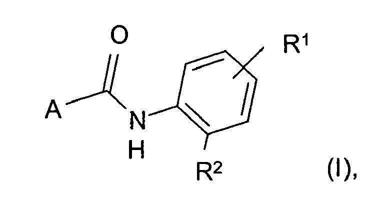 dans laquelle
R¹ représente l'hydrogène, un halogène, -CR' avec R' = l'hydrogène, le fluor ou un groupe O-(alkyle en C₁-C₄) ;
R² représente -CH(Me)-CH₂-CHMe₂, -CH₂-CH₂-t-But ou 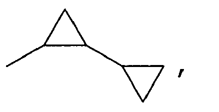
A représente le reste de la formule (A1), 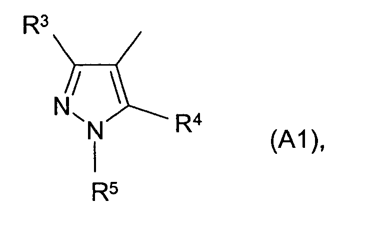
dans laquelle
R³ représente l'hydrogène, un groupe cyano, un atome d'halogène, un groupe nitro, alkyle en C₁-C₄, alcoxy en C₁-C₄, alkylthio en C₁-C₄, cycloalkyle en C₃-C₆, halogénoalkyle en C₁-C₄, halogénoalcoxy en C₁-C₄ ou halogénoalkylthio en C₁-C₄ avec à chaque fois de 1 à 5 atomes d'halogène, aminocarbonyle ou aminocarbonyl-(alkyle en C₁-C₄) ;
R⁴ représente l'hydrogène, un halogène, un groupe cyano, alkyle en C₁-C₄, alcoxy en C₁-C₄ ou alkylthio en C₁-C₄ ;
R⁵ représente l'hydrogène, un groupe alkyle en C₁-C₄, hydroxyalkyle en C₁-C₄, alcényle en C₂-C₆, cycloalkyle en C₃-C₆, (alkyle en C₁-C₄)-thio-(alkyle en C₁-C₄), (alcoxy en C₁-C₄)-(alkyle en C₁-C₄), halogénoalkyle en C₁-C₄, (halogénoalkyle en C₁-C₄)-thio-(alkyle en C₁-C₄), (halogénoalcoxy en C₁-C₄)-(alkyle en C₁-C₄) avec à chaque fois de 1 à 5 atomes d'halogène, ou phényle ;
ou
A représente le reste de la formule (A2); 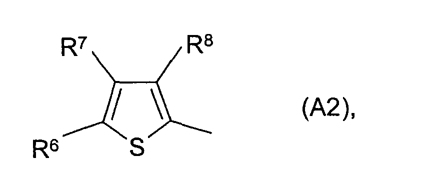
dans laquelle
R⁶ et R⁷ représentent indépendamment l'un de l'autre l'hydrogène, un halogène, un groupe alkyle en C₁-C₄ ou halogénoalkyle en C₁-C₄ avec de 1 à 5 atomes d'halogène,
R⁸ représente un halogène, un groupe cyano ou alkyle en C₁-C₄ ou halogénoalkyle en C₁-C₄ ou halogénoalcoxy en C₁-C₄ avec à chaque fois de 1 à 5 atomes d'halogène,
ou
A représente le reste de la formule (A3), 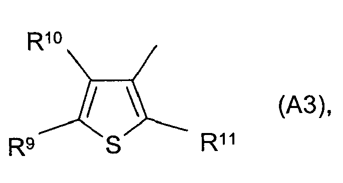
dans laquelle
R⁹ et R¹⁰ représentent indépendamment l'un de l'autre l'hydrogène, un halogène, un groupe alkyle en C₁-C₄ ou halogénoalkyle en C₁-C₄ avec de 1 à 5 atomes d'halogène,
R¹¹ représente l'hydrogène, un halogène, un groupe alkyle en C₁-C₄ ou halogénoalkyle en C₁-C₄ avec de 1 à 5 atomes d'halogène,
ou
A représente le reste de la formule (A4), 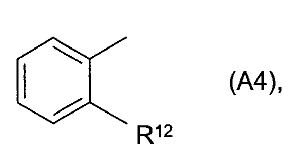
dans laquelle
R¹² représente l'hydrogène, un halogène, un groupe hydroxy, cyano, alkyle en C₁-C₆, halogénoalkyle en C₁-C₄, halogénoalcoxy en C₁-C₄ ou halogénoalkylthio en C₁-C₄ avec à chaque fois de 1 à 5 atomes d'halogène,
ou
A représente le reste de la formule (A5), 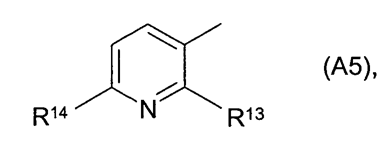
dans laquelle
R¹³ représente un halogène, un groupe hydroxy, cyano, alkyle en C₁-C₄, alcoxy en C₁-C₄, alkylthio en C₁-C₄, halogénoalkyle en C₁-C₄, halogénoalkylthio en C₁-C₄ ou halogénoalcoxy en C₁-C₄ avec à chaque fois de 1 à 5 atomes d'halogène,
R¹⁴ représente l'hydrogène, un halogène, un groupe cyano, alkyle en C₁-C₄, alcoxy en C₁-C₄, alkylthio en C₁-C₄, halogénoalkyle en C₁-C₄, halogénoalcoxy en C₁-C₄ avec à chaque fois de 1 à 5 atomes d'halogène, alkylsulfinyle en C₁-C₄ ou alkylsulfonyle en C₁-C₄,
ou
A représente le reste de la formule (A6) 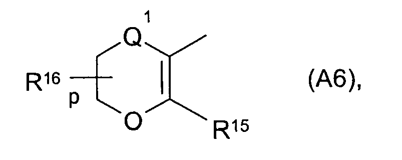
dans laquelle
R¹⁵ représente un groupe alkyle en C₁-C₄ ou halogénoalkyle en C₁-C₄ avec de 1 à 5 atomes d'halogène,
R¹⁶ représente un groupe alkyle en C₁-C₄,
Q¹ représente S (soufre), O (oxygène), SO, SO₂ ou CH₂,
p représente 0, 1 ou 2, les R¹⁶ étant des restes identiques ou différents lorsque p est égal à 2,
ou
A représente le reste de la formule (A7) 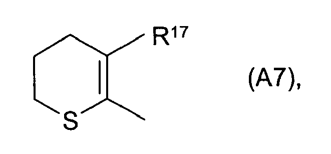
dans laquelle
R¹⁷ représente un groupe alkyle en C₁-C₄ ou halogénoalkyle en C₁-C₄ avec de 1 à 5 atomes d'halogène,
ou
A représente le reste de la formule (A8) 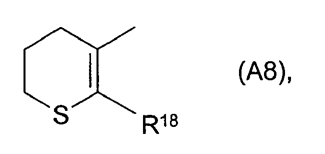 dans laquelle
R¹⁸ représente un groupe alkyle en C₁-C:₄ ou halogénoalkyle en C₁-C₄ avec de 1 à 5 atomes d'halogène,
ou
A représente le reste de la formule (A9) 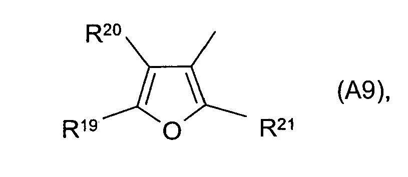
dans laquelle
R¹⁹ et R²⁰ représentent indépendamment l'un de l'autre l'hydrogène, un halogène, un groupe amino, alkyle en C₁-C₄ ou halogénoalkyle en C₁-C₄ avec de 1 à 5 atomes d'halogène,
R²¹ représente l'hydrogène, un halogène, un groupe alkyle en C₁-C₄ ou halogénoalkyle en C₁-C₄ avec de 1 à 5 atomes d'halogène,
ou
A représente le reste de la formule (A10) 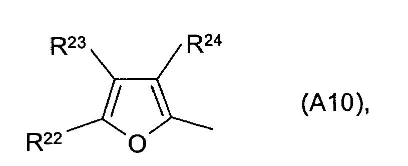
dans laquelle
R²² et R²³ représentent indépendamment l'un de l'autre l'hydrogène, un halogène, un groupe amino, nitro, alkyle en C₁-C₄ ou halogénoalkyle en C₁-C₄ avec de 1 à 5 atomes d'halogène,
R²⁴ représente l'hydrogène, un halogène, un groupe alkyle en C₁-C₄ ou halogénoalkyle en C₁-C₄ avec de 1 à 5 atomes d'halogène,
ou
A représente le reste de la formule (A11) 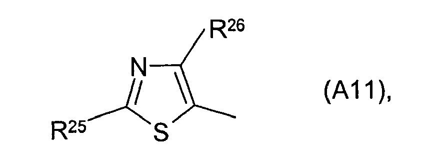
dans laquelle
R²⁵ représente l'hydrogène, un halogène, un groupe amino, alkylamino en C₁-C₄, di-(alkyle en C₁-C₄)amino, cyano, alkyle en C₁-C₄ ou halogénoalkyle en C₁-C₄ avec de 1 à 5 atomes d'halogène,
R²⁶ représente un halogène, un groupe alkyle en C₁-C₄ ou halogénoalkyle en C₁-C₄ avec de 1 à 5 atomes d'halogène,
ou
A représente le reste de la formule (A12) 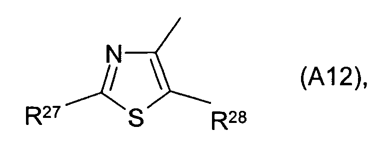
dans laquelle
R²⁷ représente l'hydrogène, un halogène, un groupe amino, alkylamino en C₁-C₄, di-(alkyle en C₁-C₄)amino, cyano, alkyle en C₁-C₄ ou halogénoalkyle en C₁-C₄ avec de 1 à 5 atomes d'halogène,
R²⁸ représente un halogène, un groupe alkyle en C₁-C₄ ou halogéno-alkyle en C₁-C₄ avec de 1 à 5 atomes d'halogène,
ou
A représente le reste de la formule (A13) 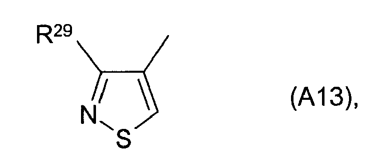
dans laquelle
R²⁹ représente un halogène, un groupe alkyle en C₁-C₄ ou halogénoalkyle en C₁-C₄ avec de 1 à 5 atomes d'halogène,
ou
A représente le reste de la formule (A14) 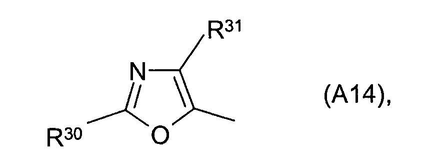 dans laquelle
R³⁰ représente l'hydrogène ou un groupe alkyle en C₁-C₄,
R³¹ représente un halogène ou un groupe alkyle en C₁-C₄,
ou
A représente le reste de la formule (A15) 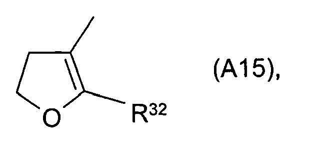
dans laquelle
R³² représente un groupe alkyle en C₁-C₄ ou halogénoalkyle en C₁-C₄ avec de 1 à 5 atomes d'halogène,
ou
A représente le reste de la formule (A16) 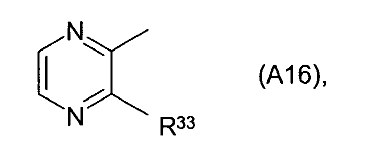 dans laquelle
R³³ représente l'hydrogène, un halogène, un groupe alkyle en C₁-C₄ ou halogénoalkyle en C₁-C₄ avec de 1 à 5 atomes d'halogène,
ou
A représente le reste de la formule (A17) 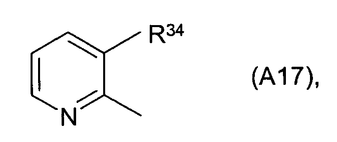
dans laquelle
R³⁴ représente un halogène, un groupe hydroxy, alkyle en C₁-C₄, alcoxy en C₁-C₄, alkylthio en C₁-C₄, halogénoalkyle en C₁-C₄, halogénoalkylthio en C₁-C₄ ou halogénoalcoxy en C₁-C₄ avec à chaque fois de 1 à 5 atomes d'halogène,
ou
A représente le reste de la formule (A18) 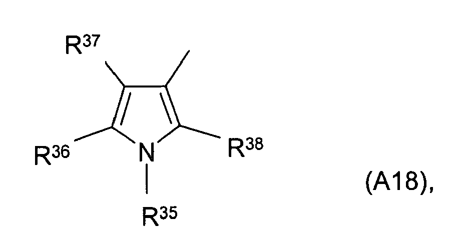
dans laquelle
R³⁵ représente l'hydrogène, un groupe cyano, alkyle en C₁-C₄, halogénoalkyle en C₁-C₄ avec de 1 à 5 atomes d'halogène, (alcoxy en C₁-C₄)-(alkyle en C₁-C₄), hydroxyalkyle en C₁-C₄, alkylsulfonyle en C₁-C₄, di(alkyle en C₁-C₄)aminosulfonyle, alkylcarbonyle en C₁-C₆ ou phénylsulfonyle ou benzoyle à chaque fois éventuellement substitué,
R³⁶ représente l'hydrogène, un halogène, un groupe alkyle en C₁-C₄ ou halogénoalkyle en C₁-C₄ avec de 1 à 5 atomes d'halogène,
R³⁷ représente l'hydrogène, un halogène, un groupe cyano, alkyle en C₁-C₄ ou halogénoalkyle en C₁-C₄ avec de 1 à 5 atomes d'halogène,
R³⁸ représente l'hydrogène, un halogène, un groupe alkyle en C₁-C₄ ou halogénoalkyle en C₁-C₄ avec de 1 à 5 atomes d'halogène,
ou
A représente le reste de la formule (A19) 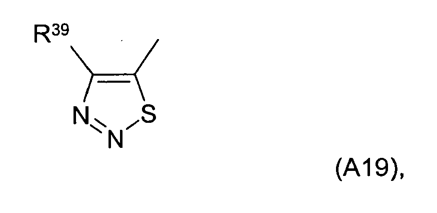 dans laquelle
R³⁹ représente un groupe alkyle en C₁-C₄,
**caractérisé en ce que** l'on fait réagir
dans une première étape un amide d'acide carboxylique de la formule (II) 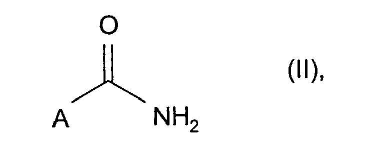 dans laquelle le reste A a la signification indiquée ci-dessus
avec des halogénoalkylbenzènes de la formule (III) 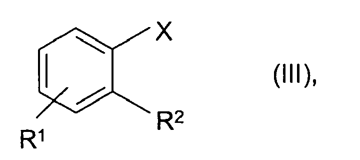 dans laquelle les restes R¹ et R² ont les significations indiquées ci-dessus et le reste X est un atome d'halogène,
dans une réaction métal-catalysée.

2. Procédé selon la revendication 1, dans lequel les amides d'acides carboxyliques de la formule (II), lesquels sont choisis dans le groupe constitué des composés des formules (IV) à (VI) 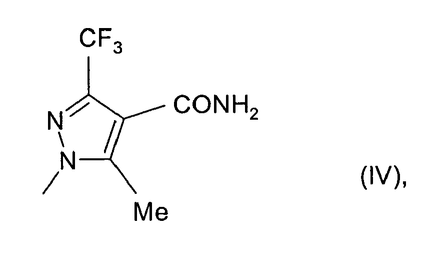 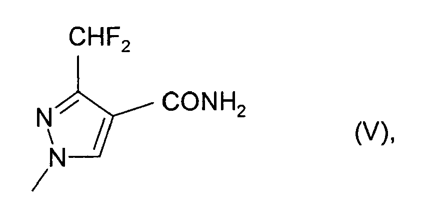 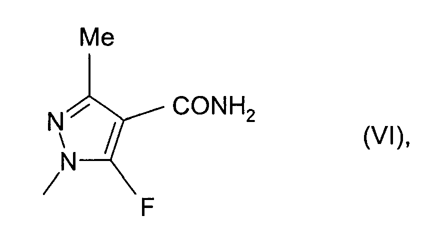 sont transformés en carboxamides selon la formule générale (VIII) 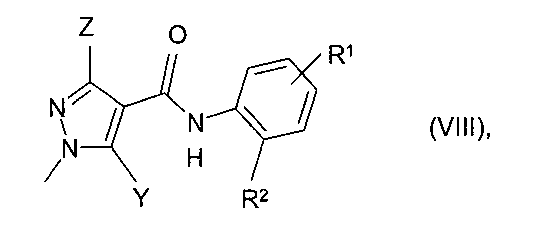 dans laquelle les restes R¹ et R² ont la signification définie dans la revendication 1,
Z représente le groupe méthyle, difluorométhyle ou trifluorométhyle et
Y représente l'hydrogène, le groupe méthyle ou le fluor.

3. Procédé selon l'une quelconque des revendications 1 ou 2, **caractérisé en ce que** l'on fait réagir des amides d'acides carboxyliques selon la formule (V) 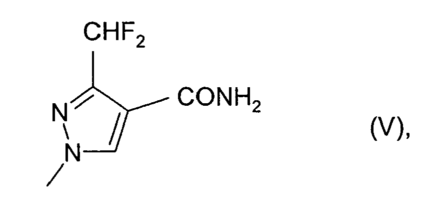 avec des composés de la formule (X) 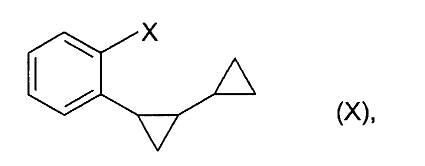 dans laquelle le reste X représente un atome d'halogène,
en un carboxamide de la formule (XI)

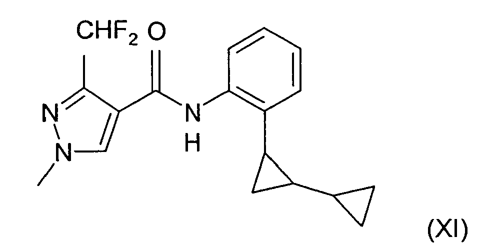

4. Procédé selon l'une quelconque des revendications 1 à 3, **caractérisé en ce que** le catalyseur de métal est choisi parmi les catalyseurs de palladium ou de cuivre.

5. Procédé selon l'une quelconque des revendications 1 à 4, **caractérisé en ce que** l'on utilise au moins un composé contenant du cuivre dans des quantités catalytiques en présence d'un ligand.

6. Procédé selon l'une quelconque des revendications 1 à 5, avec l'utilisation d'une base.

7. Procédé selon la revendication 5, dans' lequel on utilise comme ligand des diamines.

8. Composé de la formule (III) 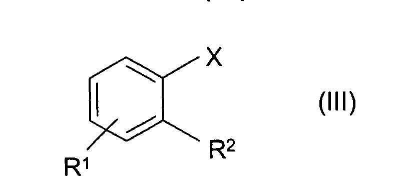 dans laquelle
R¹ représente l'hydrogène, un halogène, -CR'(CF₃)₂ avec R' l'hydrogène, le fluor ou un groupe O-(alkyle en C₁-C₄), et
R² représente -CH(Me)-CH₂-CHMe₂, -CH₂-CH₂-t-But
ou
R¹ représente un halogène, -CR'(CF₃)₂ avec R' = l'hydrogène, le fluor ou un groupe O-(alkyle en C₁-C₄), et
R² représente 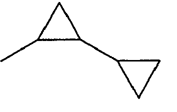
et
X représente un atome d'halogène.

9. Composé selon la revendication 8, de la formule (IX) 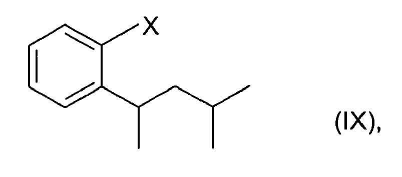 dans laquelle
X représente un atome d'halogène.

10. Composés des formules (IV) à (VI)

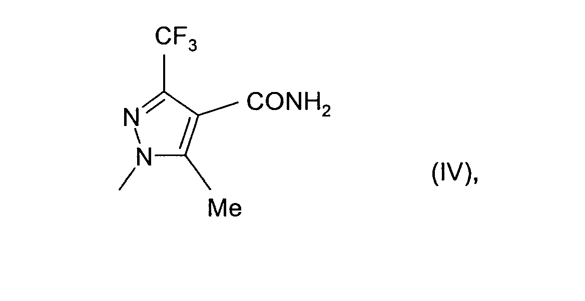

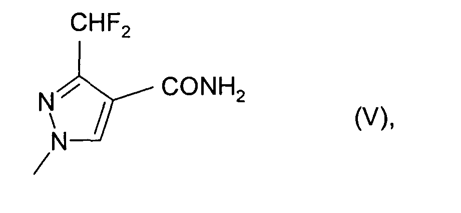

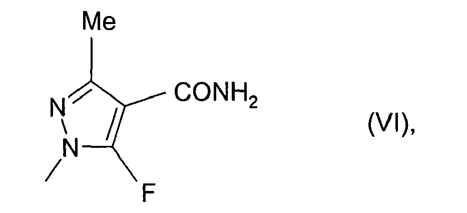

11. Utilisation d'au moins un composé selon au moins l'une des revendications 8 à 10 dans un procédé selon l'une des revendications 1 à 7.
